(19) **Europäisches Patentamt**

**European Patent Office**

**Office européen des brevets**

(11) **EP 0 737 080 B1**

(12) **FASCICULE DE BREVET EUROPEEN**

(45) Date de publication et mention
de la délivrance du brevet:
**10.11.1999 Bulletin 1999/45**

(21) Numéro de dépôt: **95905683.9**

(22) Date de dépôt: **28.12.1994**

(51) Int. Cl.$^6$: **A61M 5/172**

(86) Numéro de dépôt international:
**PCT/FR94/01549**

(87) Numéro de publication internationale:
**WO 95/17914 (06.07.1995 Gazette 1995/29)**

(54) **PROCEDE D'INJECTION CONTROLLEE PAR UNE POMPE A PERFUSION**

DURCH EINE INFUSIONSPUMPE GEREGELTES INJEKTIONSVERFAHREN

METHOD OF INJECTION CONTROLLED BY AN INFUSION PUMP

(84) Etats contractants désignés:
**AT BE CH DE DK ES FR GB GR IE IT LI LU NL PT
SE**

(30) Priorité: **29.12.1993 FR 9316022**

(43) Date de publication de la demande:
**16.10.1996 Bulletin 1996/42**

(73) Titulaire: **ZAMBON GROUP S.p.A.
I-20091 Bresso-Milano (IT)**

(72) Inventeur:
**VIALLET, Fabien,
Résidence Henri-Matisse Bat B
F-06140 Vence (FR)**

(74) Mandataire: **Bonneau, Gérard
Cabinet Bonneau,
Conseil en Propriété Industrielle,
7, Avenue Gazan
06600 Antibes (FR)**

(56) Documents cités:
**EP-A- 0 039 044       EP-A- 0 285 403
US-A- 4 367 435**

## Description

Domaine technique

La présente invention concerne un procédé d'injection contrôlée de liquide dans un tube utilisé dans un système du type comprenant une source de liquide à injecter reliée au tube en amont de celui-ci, une pompe agissant par pression exercée sur le tube pour injecter le liquide en provenance de la source de liquide vers l'aval du tube, un moteur rotatif à courant continu pour actionner la pompe et obtenir une quantité de liquide injecté fonction de l'angle dont a tourné le moteur, et une unité de commande du moteur, le moteur transmettant à l'unité de commande des impulsions dont la fréquence est proportionnelle à la vitesse du moteur.

Etat de la technique

De plus en plus de maladies humaines sont traitées par injection d'une substance médicamenteuse dans le corps du patient. Ainsi, dans le traitement du diabète, il est nécessaire de pratiquer des injections régulières d'insuline au patient. D'autres maladies telles que le cancer sont également traitées par injection de substances médicamenteuses. Mais les injections régulières par aiguille et seringue posent de nombreux problèmes tels que la régularité des injections à respecter, et surtout le problème posé par les dommages cutanés occasionnés par les multiples injections effectuées.

La solution a donc été pour le patient d'avoir un traitement continu grâce à une aiguille à perfusion placée à demeure à l'entrée du cathéter implanté chez le patient, en général au moyen d'une chambre implantée, connectée à une pompe.

L'amélioration apportée par cette technique a été que le patient porte la pompe sur lui, dans une poche, ou accrochée à une ceinture. La pompe entraînée par un petit moteur électrique, injecte de façon continue la substance médicamenteuse dans le cathéter, et donc fournit la chimiothérapie nécessaire au patient sans avoir à pratiquer de continuelles injections par aiguille et seringue.

Quel que soit le système mécanique utilisé par les pompes d'injection d'une substance médicamenteuse (péristaltique, nutation, doigts, cames...), une injection en continu se traduit par une fragmentation du volume à délivrer en petites quantités injectées à intervalles réguliers. Cette technique de fragmentation est nécessaire car les volumes injectés sont faibles pour des durées généralement longues, surtout lorsqu'il s'agit de systèmes ambulatoires et portables. Afin de se rapprocher d'un fonctionnement continu, le processus est toujours le même: la pompe injecte dans le tube relié à l'aiguille de perfusion une quantité de substance médicamenteuse appelée bolus, toujours identique, par exemple 25 $\mu$l ou 50 $\mu$l. Seule la période de fragmentation peut-être modifiée de façon à obtenir un traitement sur une période plus ou moins longue.

La méthode ci-dessus a pour avantage de nécessiter une gestion de commande du moteur relativement simple. Mais elle présente l'inconvénient que les résultats pharmacocinétiques ne sont pas toujours adaptés au traitement. En effet, comme le bolus minimum est fixe, une injection d'un petit volume (50, 100 ml) de substance médicamenteuse devant se faire sur une durée longue (4 ou 5 jours) nécessite obligatoirement des longues périodes de fragmentation. Ainsi, si le traitement exige une injection de 100 ml pendant 5 jours, le débit théorique est de 0,83ml/h ou 13,88 $\mu$l/mn. Mais comme le système fournit un bolus de 50 $\mu$l, il faut une période de 3mn 36 sec, c'est à dire que le système délivrera 50 $\mu$l toutes les 3mn 36 sec.

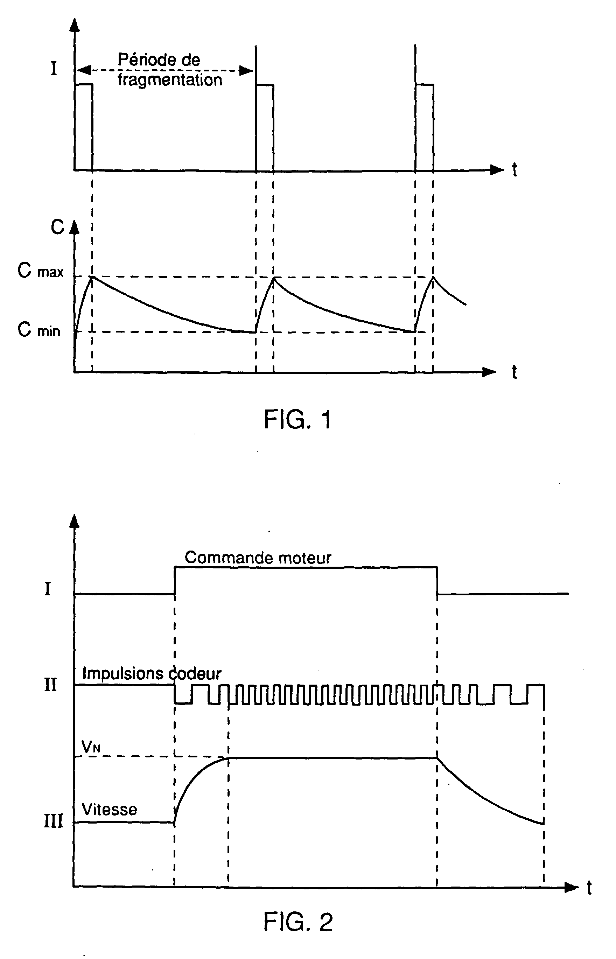

Ce type de commande ne donne pas de bons résultats d'un point de vue pharmaco-cinétique parce que la perfusion se traduit par une grande fluctuation de concentration de produit injecté entre deux injections comme l'illustre la figure 1. A chaque injection représentée sur le premier graphique par les impulsions espacées dans le temps par une période de fragmentation, la concentration de la substance médicamenteuse injectée dans l'organisme du patient augmente jusqu'à une concentration maximum Cmax. Puis après cette injection et jusqu'à la suivante, la concentration diminue jusqu'à une concentration minimum Cmin.

Afin de diminuer au maximum le rapport Cmax/ Cmin, il faut rapprocher les injections le plus possible, ce qui nécessite l'injection de boli élémentaires plus petits et une période de fragmentation plus courte. Ceci n'est réalisable qu'avec des systèmes mécaniques du "type continu" dotés d'une commande moteur élaborée afin d'obtenir des précisions acceptables. En fonction des débits demandés, il faut pouvoir faire varier les deux paramètres que sont le bolus et la période de fragmentation ou seulement le bolus si la période choisie est acceptable dans tous les cas.

Mais ce type de commande exige une grande précision à chaque injection ce qui entraîne la nécessité de disposer d'un matériel encombrant et d'un logiciel compliqué (du type PID) pour un asservissement en position et/ou en vitesse. En outre, le matériel du fait qu'il est lourd et encombrant et le logiciel du fait qu'il prend beaucoup de temps CPU, sont tous deux gros consommateurs d'énergie, ce qui n'est absolument pas adapté aux pompes ambulatoires ne disposant que de piles pour l'alimentation de l'ensemble.

C'est pourquoi on a cherché à utiliser des méthodes de perfusion à l'aide de pompes ambulatoires permettant de contrôler et de réguler l'injection du liquide en utilisant a chaque phase de fonctionnement une correction basée sur la quantité de liquide injectée pendant la phase de fonctionnement précédente. Ainsi, le brevet EP-A-285.403 décrit une pompe de perfusion mettant en oeuvre une méthode dans laquelle on compte le nombre d'impulsions lorsque le moteur est alimenté et le nombre d'impulsions après arrêt de l'alimentation jusqu'à l'arrêt complet du moteur. On additionne ces deux nombres pour comparer le nombre total d'impulsions effectivement obtenu au nombre d'impulsions théorique qui devait être obtenu lors de la phase de fonctionnement considérée. La différence est alors prise en considération lors de la phase de fonctionnement suivante. Malheureusement, cette méthode ne permet pas de réduire l'erreur absolue à zéro ou à une valeur proche de zéro du fait qu'on n'obtient pas une distribution homogène dans le temps des impulsions transmises par le moteur de la pompe.

Exposé de l'invention

Un premier but de l'invention est donc de fournir un procédé d'injection de liquide dans un tube permettant d'obtenir une distribution du liquide sensiblement homogène dans le temps.

Un autre but de l'invention est de fournir un procédé d'injection de liquide dans un tube à l'aide d'un moteur fournissant des impulsions à une pompe en utilisant à chaque phase de fonctionnement, une correction permettant de réduire l'erreur absolue à une valeur proche de zéro.

Encore un autre but de l'invention est de réaliser un procédé d'injection d'une substance médicamenteuse au moyen d'une pompe ambulatoire, qui permet d'avoir un rapport entre la concentration maximum et la concentration minimum de la substance dans l'organisme du patient très proche de un.

L'objet de l'invention est donc un procédé caractérisé par les étapes suivantes, mises en oeuvre à chaque phase n de fonctionnement du moteur: compter les impulsions transmises par le moteur et fournir un signal de coupure d'alimentation du moteur lorsque le nombre d'impulsions atteint une valeur de consigne $V_n$, compter le nombre d'impulsions m transmises par le moteur entre la coupure de son alimentation et son arrêt, faire la somme $V_n + m$ et l'ajouter à la somme totale $N_n$ des impulsions accumulées depuis la première mise en marche du moteur, soustraire de cette somme ainsi trouvée une valeur théorique égale au produit d'un nombre prédéterminé par n de façon à obtenir une erreur algébrique, et remplacer la valeur de consigne par une nouvelle valeur qui est fonction de la différence entre le nombre prédéterminé et l'erreur algébrique, de manière à ce que l'erreur algébrique soit approximativement la même à chaque phase de fonctionnement du moteur. A chaque phase de fonctionnement, cette nouvelle valeur est égale à la différence entre le nombre prédéterminé et un nombre égal à la somme de l'erreur algébrique et d'une constante prédéterminée définie comme étant le nombre moyen d'impulsions transmis par le moteur entre la coupure de son alimentation et son arrêt lorsque le nombre total d'impulsions transmises par le moteur est égal au nombre prédéterminé.

Brève description des dessins

Les buts, objets et caractéristiques de l'invention seront mieux compris à la lecture de la description qui suit faite en référence aux dessins dans lesquels

la figure 1 représente les diagrammes par rapport au temps des impulsions d'injection d'une substance médicamenteuse et de la concentration résultante de substance dans l'organisme du patient.
la figure 2 représente sur un même diagramme par rapport au temps, les courbes de commande moteur, des impulsions transmises en retour par le moteur et de vitesse du moteur,
la figure 3 représente schématiquement un premier mode de réalisation de l'invention utilisant un microcontrôleur, et
la figure 4 représente schématiquement un deuxième mode de réalisation de l'invention utilisant un microprocesseur.

Description détaillée de l'invention

Code il a été mentionné précédemment, un des buts de l'invention est d'obtenir un rapport des concentrations maximum et minimum (voir figure 1) de la substance médicamenteuse dans l'organisme du patient, qui soit le plus proche de 1.

Pour arriver à ce résultat, il est utile auparavant de comprendre le fonctionnement d'un moteur à courant continu utilisé pour la commande de la pompe en référence à la figure 2. De façon générale lorsque le moteur est dans la phase de fonctionnement représentée par la courbe 1, il transmet des impulsions de codeur à l'unité de commande.

Comme illustré par la courbe II, lorsque le moteur est mis en marche, il accélère jusqu'à atteindre sa vitesse nominale Vn au bout d'un certain temps, comme illustré sur la courbe III. Puis il reste à cette vitesse nominale jusqu'à l'arrêt de l'alimentation. A partir de cet arrêt, il décélère jusqu'à son arrêt complet. Il y a donc transmission d'impulsions par le moteur pendant toute la durée où il est commandé, mais également pendant sa phase de décélération après la coupure de l'alimentation. Pour empêcher que la concentration de la substance médicamenteuse ne descende trop bas, la logique veut que l'on diminue la période de fragmentation (voir figure 1) tout en diminuant également la quantité de substance médicamenteuse injectée à chaque mise en marche du moteur. Mais comme on vient de le voir en référence à la figure 2, le moteur présente une phase de décélération après la fin de la commande du moteur pendant laquelle la pompe continue d'injecter le médicament, et qu'il est impossible de connaître avec précision à moins de disposer de moyens très sophistiqués comme il a déjà été mentionné, incompatibles avec une pompe portable. Plus on diminue la période de fragmentation, plus on augmente la fréquence de mise en route du moteur, et plus on augmente l'erreur de précision sur la quantité injectée due aux phases de décélération du moteur.

Le principe de l'invention consiste donc à commander la durée de fonctionnement du moteur à chaque mise en route de façon à compenser l'erreur accumulée précédemment, ceci grâce à la transmission des impulsions (générées par un codeur optique ou un codeur magnétique) par le moteur vers l'unité de commande.

On commence par déterminer une période de fragmentation courte qui empêche la concentration de la substance médicamenteuse de descendre trop bas. Généralement, il faut choisir cette période bien en dessous de la demi-vie de la substance injectée. Connaissant le volume total à injecter, on détermine le volume basal ou bolus à injecter à chaque période. Ce volume basal est facilement converti en nombre d'impulsions du codeur, puisqu'on connaît le volume délivré par tour du moteur et donc le volume injecté à chaque impulsion. On connaît donc le nombre d'impulsions théoriques correspondant au fonctionnement du moteur pour chaque période de fragmentation. Le procédé va donc consister à rectifier, à chaque mise en route du moteur, le nombre d'impulsions théoriques de commande du moteur par l'erreur absolue du nombre cumulé d'impulsions, erreur due à la période de décélération du moteur dont il est impossible de déterminer la durée avec précision, de façon à obtenir une valeur de consigne (en nombre d'impulsions) de commande moteur.

L'exemple suivant permettra de mieux comprendre le principe de l'invention. Supposons que la période de fragmentation choisie pour le traitement ait permis de déterminer un nombre théorique de 10 impulsions pendant lesquelles le moteur doit être commandé à chaque phase M de fonctionnement.

Les valeurs des différentes variables (valeur de consigne, erreur relative, erreur absolues,...) pendant les phases de fonctionnement sont données dans le tableau I.

| Phase de fonctionnement | 1 | 2 | 3 | 4 | 5 | 6 | 7 | 8 |
|---|---|---|---|---|---|---|---|---|
| valeur de consigne | 10 | 4 | 7 | 6 | 6 | 7 | 6 | 6 |
| nombre d'impulsions de la phase | 16 | 7 | 11 | 10 | 9 | 11 | 10 | 10 |
| erreur relative | 6 | 3 | 4 | 4 | 3 | 4 | 4 | 4 |
| nombre d'impulsions cumulées | 16 | 23 | 34 | 44 | 53 | 64 | 74 | 84 |
| erreur absolue | 6 | 3 | 4 | 4 | 3 | 4 | 4 | 4 |

A chaque phase de fonctionnement, la valeur de la consigne est égale à 10 (nombre théorique de commande moteur) diminuée de l'erreur sur le nombre total d'impulsions souhaité pour respecter la chronothérapie. Ainsi, à la phase 2, l'erreur étant de 6, la valeur de consigne est 4. A la phase 3, l'erreur étant de 3, la valeur de consigne est établie à 7... et ainsi de suite . On voit, à la lecture du tableau ci-dessus, que l'erreur cumulée tend à se stabiliser à 4 ou plus généralement entre 3 et 5. Une telle erreur correspond en fin de traitement à quelques centaines de nanolitres, donc est négligeable comparée à 50 ou 100 ml injectés au total. On doit noter que cette erreur est directement liée au moment d'inertie du moteur donc à la vitesse atteinte par le moteur lors de la coupure d'alimentation du moteur.

A la lecture des chiffres de l'exemple ci-dessus, on voit que l'erreur absolue est en fait égale à l'erreur relative (nombre d'impulsions renvoyées par le moteur pendant la phase de décélération). Ceci est dû au fait que

Si $N_n$ est le nombre d'impulsions cumulées à la phase n, $V_n$ la valeur de consigne et $e_n$ est l'erreur relative à la phase n, le nombre d'impulsions cumulées à la phase n est égal à

$$N_n = N_{n-1} + V_n + e_n$$

et donc l'erreur absolue à la phase n est égale à

$$E_n = N_n - 10.n$$

soit

$$E_n = N_{n-1} + V_n + e_n - 10.n$$

et comme à chaque phase la nouvelle valeur de consigne est calculée en retranchant de 10 l'erreur absolue précédente , soit:

$$V_n = 10 - [N_{n-1} - 10.(n-1)]$$

$$V_n = 10.n - N_{n-1}$$

on en tire

$$E_n = e_n$$

Donc à chaque phase, l'erreur absolue est égale à l'erreur relative, c'est à dire au nombre d'impulsions de la phase de décélération du moteur.

Comme on l'a mentionné précédemment le nombre d'impulsions renvoyées par le moteur n'est pas une valeur pouvant être déterminée avec certitude. Il est toutefois possible d'en connaître la valeur approximative. C'est pourquoi le procédé qui vient d'être décrit peut être amélioré en introduisant une valeur rectificative prédéterminée dans le calcul de la valeur de consigne. La valeur de consigne est, à chaque phase, égale au nombre théorique d'impulsions diminué de l'erreur absolue précédente à laquelle on ajoute une constante prédéterminée permettant d'obtenir un nombre total d'impulsions (y compris la phase de décélération) approximativement égal au nombre théorique. Ainsi, toujours dans l'hypothèse d'une valeur théorique de 10 impulsions à chaque phase de fonctionnement, et en considérant une constante rectificative de 4, les valeurs des différentes variables (valeur de consigne, erreur relative, erreur absolue,...) pendant les phases de fonctionnement sont données dans le tableau II suivant.

| Phase de fonctionnement | 1 | 2 | 3 | 4 | 5 | 6 | 7 | 8 |
|---|---|---|---|---|---|---|---|---|
| valeur de consigne | 6 | 6 | 5 | 7 | 6 | 6 | 5 | 7 |
| nombre d'impulsions de la phase | 10 | 11 | 8 | 11 | 10 | 11 | 8 | 11 |
| erreur relative | 4 | 5 | 3 | 4 | 4 | 5 | 3 | 4 |
| nombre d'impulsions cumulées | 10 | 21 | 29 | 40 | 50 | 61 | 69 | 80 |
| erreur absolue | 0 | 1 | -1 | 0 | 0 | 1 | -1 | 0 |

Ici encore, il est intéressant de calculer l'erreur absolue pour constater qu'elle se déduit directement de l'erreur relative.

Si $N_n$ est le nombre d'impulsions cumulées à la phase n, $V_n$ la valeur de consigne et $e_n$ est l'erreur relative à la phase n, le nombre d'impulsions cumulées à la phase n est égal à

$$N_n = N_{n-1} + Vn + e_n$$

et donc l'erreur absolue à la phase n est égale à

$$E_n = N_n - 10.n$$

soit

$$E_n = N_{n-1} + V_n + e_n - 10.n$$

et comme à chaque phase la nouvelle valeur de consigne est calculée en retranchant de 10 l'erreur absolue précédente augmentée de la constante 4, soit:

$$V_n = 10 - [N_{n-1} - 10.(n-1)] - 4$$

$$V_n = 10.n - N_{n-1} - 4$$

on en tire

$$E_n = e_n - 4$$

On constate donc qu'il suffit encore de considérer l'erreur relative de la phase précédente pour calculer la valeur de consigne pour chaque phase, sauf pour la première phase où la valeur de consigne est égale au nombre d'impulsions théorique diminué de la constante prédéterminée.

Afin d'améliorer le procédé, on peut envisager d'avoir plusieurs tensions d'alimentation du moteur pouvant être sélectionnées, permettant une plus grande plage d'utilisation. En effet, plus la tension est faible, moins l'erreur est importante du fait d'une vitesse nominale plus faible.

Dans le mode de réalisation préféré de l'invention, le moteur à courant continu alimenté sous 5V commande la pompe avec une réduction de 1/27, et un codeur optique fournissant 16 impulsions per tour moteur.

Avec des caractéristiques de la pompe telles que la quantité de liquide injectée à chaque tour de pompe soit de 25µl, la quantité injectée à chaque impulsion est alors de 25µl / 27x16 = 57,87 nl.

Si on choisit une période de fragmentation de 5s, c'est à dire si le moteur est mis en fonctionnement toutes les 5s, et en prenant un nombre d'impulsions théorique égal à 10 à chaque phase de fonctionnement, la quantité de substance médicamenteuse injectée par heure sera de

$$57,87nl \times 10 \times 720 = 416,66µl$$

Avec la même pompe, mais en choisissant un nombre d'impulsions théorique égal à 20 on obtient une quantité horaire de 883,32 µl. Ainsi, avec une même période de fragmentation, mais en choisissant un nombre d'impulsions théorique différent, il est possible d'adapter la chronothérapie selon le traitement désiré.

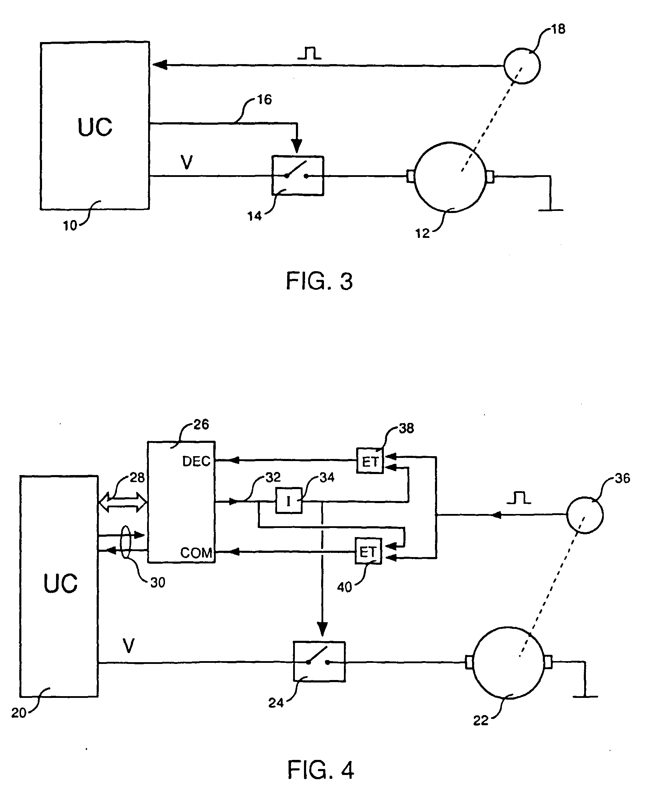

Le procédé de l'invention peut être implémenté de façon générale par le système illustré sur la figure 3. L'unité de commande est un microcontrôleur fournissant l'alimentation V au moteur 12 au moyen du commutateur 14. Le microcontrôleur comporte en mémoire la valeur du nombre d'impulsions théorique à délivrer à chaque phase de fonctionnement du moteur. Au démarrage d'une phase de fonctionnement, le microcontrôleur envoie un signal de validation sur la ligne 16 pour fermer le commutateur 14 et alimenter le moteur 12. Le codeur 18 transmet en retour des impulsions au microcontrôleur 10. Celles-ci sont décomptées de la valeur de consigne jusqu'à la valeur 0. A cette valeur, le microcontrôleur cesse de transmettre le signal de validation sur la ligne 16 et le moteur 12 n'est plus alimenté. Pendant la phase de décélération, les impulsions transmises par le codeur 18 sont comptées pour déterminer l'erreur relative qui va servir pour calculer l'erreur absolue et la nouvelle valeur de consigne à appliquer lors de la phase de fonctionnement suivante.

Le mode de réalisation préféré de l'invention peut être réalisé par le système illustré sur la figure 4. Dans cette réalisation, l'unité de commande est simplement un microprocesseur 20. De la même façon que précédemment, la tension d'alimentation est fournie au moteur 22 par l'intermédiaire d'un commutateur 24. Un compteur/décompteur 26 reçoit de/ou transmet ses données au microprocesseur 20 au moyen d'un bus 28 de 8 bits, la commande d'écriture ou de lecture se faisant au moyen des lignes de commande 30. Au démarrage d'une phase de fonctionnement du moteur, la valeur de consigne égale à un nombre d'impulsions théorique diminué d'une constante prédéterminée, est chargée dans le compteur/décompteur 26. La sortie 32 de ce dernier étant activée seulement lorsque le compteur est vide, elle est donc à 0 et un 1 est fourni à la sortie de l'inverseur 34, ce qui a pour effet de fermer le commutateur 24 permettant l'alimentation du moteur 22. Par conséquent, le codeur 36 transmet des impulsions. Du fait que la sortie de l'inverseur 34 est à 1, la porte ET 38 est passante alors que la porte ET 40 recevant un O en sortie de la ligne 32 est bloquée. Les impulsions transmises par le codeur 36 sont donc fournies, via le circuit ET 38, à l'entrée de décomptage DEC du compteur/décompteur 26. Lorsque le compteur/décompteur 26 atteint la valeur 0, un signal 1 est envoyé sur la ligne 32, qui rend passante la porte ET 40 mais bloque la porte ET 38 du fait que la sortie de l'inverseur 34 est à O. En outre, la sortie à O de l'inverseur 34 ouvre le commutateur 24 et coupe l'alimentation du moteur 22. Les impulsions fournies par le codeur 36 pendant la phase de décélération du moteur sont donc transmises, via la porte ET 40, à l'entrée de comptage COM du compteur/décompteur 26. A la fin de la phase de décélération, la valeur atteinte par le compteur 26 lue par le microprocesseur 20 au moyen du bus 28 est soustraite du nombre théorique d'impulsions (10 dans les exemples donnés ci-dessus) de façon à obtenir la nouvelle valeur de consigne à charger dans le compteur/décompteur 26 au démarrage de la phase suivante.

Le logiciel nécessaire pour implémenter le procédé de l'invention dans le système illustré sur la figure 4 est très simple. En effet, on commence par fournir les constantes du système, soit:

- le volume injecté par tour moteurs

- le nombre d'impulsions par tour moteur

A partir de ces constantes, le logiciel détermine aisément, en fonction de la durée du traitement et du volume total à délivrer pendant le traitement, le nombre d'impulsions à générer à chaque période ou valeur de consigne, soit

$$NIP = \text{nombre d'impulsions par période}$$

Le logiciel est ensuite simplement composé des instructions suivantes déroulées à chaque phase du fonctionnement du moteur

lecture du compteur $\to$ ERR
remise à zéro du compteur
calcul de NIP - ERR = VAL (valeur de consigne)
Chargement de VAL dans le décompteur

Si on diminue la valeur de consigne à la première phase par une valeur rectificative dans le but de diminuer la valeur de l'erreur comme on l'a mentionné précédemment il faut établir la valeur de débit de la variable NIP, non pas égale au nombre d'impulsions à obtenir par période, mais à ce nombre diminué de la valeur rectificative.

Afin d'améliorer le procédé mis en oeuvre par le système illustré sur la figure 4, il est possible d'adjoindre un logiciel d'auto-adaptation. En effet, du fait que les paramètres peuvent être différents suivant l'application (gros débit, petit débit...) et que les composants utilisés (tube silicone, moteur, rapport de réduction...) peuvent être différents, on obtient une réponse du moteur représentée par un nombre d'impulsions après la coupure de l'alimentation qui est différente selon les cas.

Le logiciel d'auto-adaptation comprend d'abord une séquence d'apprentissage au cours de laquelle on détermine le nombre d'impulsions généré après la coupure d'alimentation du moteur pour des valeurs de consigne progressives.

| Valeur de consigne | Nombre d'impulsions pendant la décélération |
|---|---|
| 1 impulsion | $n_1$ |
| 10 impulsions | $n_{10}$ |
| 50 impulsions | $n_{50}$ |
| 100 impulsions | $n_{100}$ |
| 200 impulsions | $n_{200}$ |
| 500 impulsions | $n_{500}$ |

On détermine la valeur $n_i$ pour un nombre i d'impulsions suffisant pour que le moteur atteigne sa vitesse nominale. Le nombre d'impulsions maximum peut être 200, 500 ou même 1000.

Ensuite, à partir des valeurs $n_1$, $n_{10}$, $n_{50}$... le logiciel fait une interpolation linéaire pour chaque couple de 2 valeurs de consigne successives de façon à déterminer un nombre n pour une valeur de consigne quelconque.

Les valeurs $n_i$ qui ont été ainsi déterminées sont utilisées lors d'une perfusion pour déterminer la constante à retrancher de la valeur de consigne théorique pour en tirer la valeur de consigne réelle à appliquer à chaque phase de fonctionnement du moteur.

Mais, on peut également prévoir une troisième phase du logiciel d'auto-adaptation qui consiste à établir une table dans laquelle on détermine la valeur de consigne à appliquer en retranchant de la valeur de consigne théorique le nombre d'impulsions correspondant obtenu à la séquence d'apprentissage du logiciel. Ainsi, la table obtenue contiendra la valeur de l'erreur relative à appliquer pour chaque valeur de consigne théorique telle qu'elle apparaît dans le tableau II ci-dessus.

Bien que dans le mode de réalisation préféré, le procédé de l'invention soit implémenté en partie au moyen d'un logiciel (dans le microprocesseur de la figure 4) et en partie au moyen de matériel, il est à la portée de l'homme du métier d'implémenter ce procédé seulement au moyen de circuits logiques. Mais avec les progrès de la miniaturisation des dispositifs à semiconducteurs, il est plus judicieux d'utiliser la puissance d'un microprocesseur pour effectuer certaines fonctions à l'aide d'un logiciel.

**Revendications**

1. Procédé d'injection contrôlée de liquide dans un tube dans un système d'injection du type comprenant une source du liquide à injecter reliée audit tube en amont de celui-ci, une pompe du type péristaltique agissant par pression sur le tube pour injecter le liquide en provenance de la source de liquide vers l'aval dudit tube, un moteur rotatif à courant continu (12, 22) pour actionner ladite pompe durant des phases de fonctionnement successives et obtenir une quantité de liquide injecté dans ledit tube qui est fonction de l'angle dont a tourné ledit moteur, et une unité de commande (10, 20) du moteur, ledit moteur transmettant à ladite unité commande des impulsions dont la fréquence est proportionnelle à la vitesse dudit moteur ; ledit procédé comprenant les étapes suivantes effectuées par ladite unité de commande à chaque phase n de fonctionnement dudit moteur:

   compter les impulsions transmises par ledit moteur et fournir un signal de coupure d'alimentation dudit moteur lorsque le nombre d'impulsions atteint une valeur de consigne $V_n$,
   compter le nombre d'impulsions m transmises par ledit moteur entre la coupure de son alimentation et son arrêt,
   faire la somme $V_n + m$ et l'ajouter à la somme totale $N_n$ des impulsions accumulées depuis la première mise en marche du moteur,
   soustraire de cette somme $N_n$ ainsi trouvée une valeur théorique égale au produit d'un nombre prédéterminé par n de façon à obtenir une erreur algébrique, et
   remplacer la valeur de consigne par une nouvelle valeur qui est fonction de la différence entre ledit nombre prédéterminé et ladite erreur algébrique, de manière à ce que ladite erreur algébrique soit approximativement la même à chaque phase de fonctionnement dudit moteur ;
   ledit procédé étant caractérisé en ce qu'à chaque phase de fonctionnement, ladite nouvelle valeur est égale à la différence entre ledit nombre prédéterminé et un nombre égal à la somme de ladite erreur algébrique et d'une constante prédéterminée définie comme étant le nombre moyen d'impulsions transmis par ledit moteur (12, 22) entre la coupure de son alimentation et son arrêt lorsque le nombre total d'impulsions transmises par ledit moteur est égal audit nombre prédéterminé.

2. Procédé selon la revendication 1 dans lequel, à la première mise en route dudit moteur (12, 22), ladite valeur de consigne m est établie à une valeur égale audit nombre prédéterminé moins ladite constante prédéterminée.

3. Procédé selon la revendication 1 ou 2, dans lequel ladite constante prédéterminée est déterminée automatiquement et préalablement à l'injection du liquide, par ladite unité de commande (10, 20) tenant compte des paramètres de l'injection à effectuer et des caractéristiques de ladite pompe et dudit moteur rotatif au moment de ladite injection.

4. Procédé selon la revendication 3, dans lequel ladite unité de commande (10, 20) comporte un logiciel d'auto-adaptation déterminant au cours d'une séquence d'apprentissage préalable à la perfusion, le nombre d'impulsions transmises par ledit moteur entre la coupure de son alimentation et son arrêt pour toutes les valeurs de consigne théoriques possibles.

5. Système de perfusion destiné à injecter une substance médicamenteuse dans un cathéter implanté dans le corps d'un patient, à l'aide d'une pompe agissant par pression sur un tube relié d'une part à un réservoir de ladite substance médicamenteuse et d'autre part audit cathéter, ladite pompe étant entraînée par un moteur (12, 22)) à courant continu pour obtenir une quantité de liquide injectée dans le catheter qui est fonction de l'angle dont à tourner le moteur, sous la commande d'une unité de commande (10, 20), ledit moteur étant associé à un codeur (18, 36) transmettant des impulsions, dont la fréquence est proportionnelle à la vitesse dudit moteur à ladite unité de commande pendant le fonctionnement du moteur; ledit système étant caractérisé par des moyens pour mettre en oeuvre le procédé de mise en oeuvre selon l'une des revendications 1 à 4.

6. Système de perfusion selon la revendication 5, dans lequel ladite unité de commande comprend un microprocesseur (20) et un compteur/décompteur (26) chargé au démarrage dudit moteur (22), par une valeur de consigne, ledit compteur/décompteur décomptant les impulsions transmises par ledit codeur (36) jusqu'à ce que son contenu atteigne 0 de façon à fournir un signal de coupure de l'alimentation dudit moteur, ledit compteur/décompteur 26 comptant alors les impulsions fournies par ledit codeur de façon à obtenir une erreur à soustraire de ladite valeur de consigne pour obtenir une nouvelle valeur de consigne à utiliser lors du démarrage suivant dudit moteur.

**7.** Système selon l'une des revendications 5, ou 6, dans lequel ladite pompe est une pompe péristaltique.

**Claims**

**1.** A method for the controlled injection of liquid in a tube in an injection system of the kind which comprises a source of liquid to be injected connected to said tube upstream from the latter, a pump of the peristaltic type acting under pressure on the tube in order to inject the liquid coming from the source of liquid downstream from said tube, a continuous current rotary motor (12, 22) to actuate said pump during successive operating phases and obtain a quantity of liquid injected in said tube which is a function of the angle at which said motor has rotated and a motor control unit (10, 20), said motor transmitting to said control unit pulses whose frequency is proportional to the speed of said motor, said method comprising the following phases, carried out by said control unit upon each operating phase n of said motor :

    counting the pulses transmitted by said motor and providing a supply cut-off signal of said motor when the number of pulses reaches a warning value Vn,
    counting the pulses, m, transmitted by said motor between the supply cut-off and its outage,
    summing Vn + m and add to this sum the total sum Nn of the pulses accumulated since the first start-up of the motor,
    subtracting from this sum Nn thus found a theoretical value equal to the product of a predetermined number by n in order to obtain an algebraic error, and
    replacing the warning value by a new value which is function of the difference between said predetermined number and said algebraic error, so that said algebraic error is approximately the same at each operating phase of said motor,
    said method being characterised in that at each operating phase said new value is equal to the difference between said predetermined number and a number equal to the sum of said algebraic error and a predetermined constant defined as being the mean number of pulses transmitted by said motor (12, 22) between supply cut-off and outage when the total number of pulses transmitted by said motor is equal to said predetermined number.

**2.** The method according to claim 1, in which upon initial start-up of said motor (12 22) said warning value, Vn, is established at a value equal to said predetermined number less said predetermined constant.

**3.** The method according to claim 1 or 2, in which said predetermined constant is determined automatically and prior to the injection of the liquid, by said control unit (10, 20), account taken of the injection parameters to be carried out and the characteristics of said pump and said rotary motor at the time of said injection.

**4.** The method according to claim 3 in which said control unit (10, 20) comprises a self-adaptation software determining, during the course of a learning sequence prior to infusion, the number of pulses transmitted by said motor between supply cut-off and outage for all possible theoretical warning values.

**5.** Infusion system designed to inject a medicamentous substance in a catheter implanted in a patient's body by means of a pump acting under pressure on a tube connected on the one hand to a reservoir of said medicamentous substance and on the other hand to said catheter, said pump being actuated by a continuous current motor (12, 22) to obtain a quantity of liquid injected in the catheter which is function of the angle at which the motor has rotated, controlled by a control unit (10, 20), said motor being associated to a coder (18, 36) which transmits pulses the frequency of which is proportional to the speed of said motor to said control unit during motor operation; said system being characterised by means for implementing said method according to any one of claims 1 to 4.

**6.** Infusion system according to claim 5, in which said control unit comprises a microprocessor (20) and a counter-discounter (26) responsible for the start-up of said motor (22), using a warning value, said counter/discounter discounting the pulses transmitted by said coder (36) until its contents reach 0 in order to provide a supply cut-off signal of said motor, said counter/discounter (26) then counting the pulses provided by said coder so as to obtain an error to be subtracted from said warning value, in order to obtain a new warning value to be used at the time of the following start-up of said motor.

**7.** System according to any one of claims 5 or 6, in which said pump is a peristaltic pump.

**Patentansprüche**

1. Verfahren zum gesteuerten Injizieren von Flüssigkeit in einen Schlauch in einem Injektionssystem, das folgendes aufweist: eine Quelle für die zu injizierende Flüssigkeit, die mit dem Schlauch eingangsseitig verbunden ist, eine peristaltische Pumpe, die durch Druck auf den Schlauch einwirkt, um die von der Flüssigkeitsquelle kommende Flüssigkeit auf die Ausgangsseite des Schlauchs zu zu injizieren, einen rotierenden Gleichstrommotor (12, 22) zum Betätigen der Pumpe während der aufeinanderfolgenden Betriebsphasen und zum Erhalten einer Menge von in den Schlauch injizierter Flüssigkeit, die von dem Winkel abhängig ist, um den sich der Motor gedreht hat, und eine Einheit (10, 20) zur Steuerung des Motors, wobei dieser Motor auf die Steuereinheit Impulse überträgt, deren Frequenz zu seiner Geschwindigkeit proportional ist, wobei dieses Verfahren aus den folgenden Schritten besteht, die von der Steuereinheit bei jeder Betriebsphase n des Motors ausgeführt werden:

   die vom Motor übertragenen Impulse zählen und ein Signal zur Unterbrechung der Versorgung des Motors liefern, wenn die Impulszahl einen Sollwert $V_n$ erreicht,
   die Anzahl m der vom Motor zwischen der Unterbrechung seiner Versorgung und seinem Stillstand übertragenen Impulse zählen,
   die Summe $V_n + m$ bilden und sie zur Gesamtsumme $N_n$ der seit der ersten Inbetriebnahme des Motors angesammelten Impulse addieren,
   von dieser so gefundenen Summe $N_n$ einen theoretischen Wert abziehen, der gleich dem Produkt aus einer vorbestimmten Zahl und n ist, so daß man einen algebraischen Fehler erhält, und
   den Sollwert durch einen neuen Wert ersetzen, der von der Differenz zwischen dieser vorbestimmten Zahl und dem algebraischen Fehler abhängig ist, so daß dieser algebraische Fehler bei jeder Betriebsphase des Motors annähernd derselbe ist,
   dadurch gekennzeichnet, daß dieser neue Wert bei jeder Betriebsphase gleich der Differenz zwischen dieser vorbestimmten Zahl und einer Zahl ist, die gleich der Summe des algebraischen Fehlers und einer vorbestimmten Konstante ist, die definiert ist als die mittlere Anzahl von Impulsen, die von dem Motor (12, 22) zwischen der Unterbrechung seiner Versorgung und seinem Stillstand übertragen wurde, wenn die Gesamtzahl vom Motor übertragener Impulse gleich der vorbestimmten Zahl ist.

2. Verfahren nach Anspruch 1, bei dem der Sollwert m bei der ersten Ingangsetzung des Motors (12, 22) auf einen Wert eingestellt wird, der gleich dieser vorbestimmten Zahl abzuglich der vorbestimmten Konstante ist.

3. Verfahren nach Anspruch 1 oder 2, bei dem die vorbestimmte Konstante durch die Steuereinheit (10, 20) vor der Injektion der Flüssigkeit automatisch bestimmt wird, wobei die Parameter der durchzuführenden Injektion und die Merkmale der Pumpe und des rotierenden Motors zum Zeitpunkt der Injektion berücksichtigt werden.

4. Verfahren nach Anspruch 3, bei dem die Steuereinheit (10, 20) eine Selbstanpassungssoftware besitzt, die während einer der Infusion vorangehenden Lernsequenz die Anzahl von Impulsen, die vom Motor zwischen der Unterbrechung seiner Versorgung und seinem Stillstand übertragen werden, für alle möglichen theoretischen Sollwerten bestimmt.

5. Infusionssystem zum Injizieren einer Arzneimittelsubstanz in einen in den Körper eines Patienten implantierten Katheter mit Hilfe einer Pumpe, die durch Druck auf einen Schlauch wirkt, der einerseits mit einem die Arzneimittelsubstanz enthaltenden Behälter und andererseits mit dem Katheter verbunden ist, und die durch einen Gleichstrommotor (12, 22) angetrieben wird, um eine in den Katheter injizierte Flüssigkeitsmenge zu erhalten, die von dem Winkel abhängig ist, um den sich der Motor gedreht hat, und zwar unter der Steuerung durch eine Steuereinheit (10, 20), wobei dem Motor ein Kodierer (18, 36) zugeordnet ist, der während des Betriebs des Motors auf die Steuereinheit Impulse überträgt, deren Frequenz zur Geschwindigkeit des Motors proportional ist, gekennzeichnet durch Mittel zur Durchführung des Verfahrens nach einem der Ansprüche 1 bis 4.

6. Infusionssystem nach Anspruch 5, in dem die Steuereinheit einen Mikroprozessor (20) und einen Vorwärts-Rückwärts-Zähler (26) aufweist, der bei Anlaufen des Motors (22) mit einem Sollwert geladen wird und der die vom Kodierer (36) übertragenen Impulse zurückzählt, bis sein Inhalt 0 erreicht, so daß ein Signal zur Unterbrechung der Versorgung des Motors geliefert wird, wobei der Vorwärts-Rückwärts-Zähler (26) die vom Kodierer gelieferten Impulse zählt, so daß man einen Fehler erhält, der vom Sollwert abzuziehen ist, um einen neuen Sollwert zu erhalten, der bei dem folgenden Anlauf des Motors verwendet wird.

7. System nach einem der Ansprüche 5 oder 6, in dem die Pumpe eine peristaltische Pumpe ist.

FIG. 1

FIG. 2

FIG. 3

FIG. 4